# EUROPEAN PATENT APPLICATION

(11) **EP 4 033 493 A1**
(43) Date of publication of application: **27.07.2022**
(21) Application number: 21153403.7
(22) Date of filing: 26.01.2021
(51) Int. Cl.: G16H 15/00, G16H 10/00, G16H 70/20

(54) **METHOD OF AUTOMATICALLY MATCHING PROCEDURE DEFINITIONS IN DIFFERENT RADIOLOGY INFORMATION SYSTEMS**

(71) Applicant: AGFA HEALTHCARE NV, 2640 Mortsel (BE)
(72) Inventor: ADRIAENSENS, Roel, 2640 Mortsel (BE); DE WITTE, Yoni, 2640 Mortsel (BE)
(74) Representative: Verbrugghe, Anne Marie L.

(57) **Abstract**

Method of automatically matching procedure definitions in different radiology information systems.

A computer-implemented method which, given a set of procedure definitions in a first radiology information system generates the best match for a procedure definition defined in a second system on the basis of a multidimensional vector representation of procedure definitions and a matching algorithm based on vector cosine similarity.

## Description

### FIELD OF THE INVENTION

The present invention is in the field of medical imaging, more particularly in the field of Radiology Information Systems (RIS).

The invention more specifically relates to a method of automatically matching procedure definitions in a format as used in a first radiology information system, e.g. the system of a client to the format in which the procedure definition is known in a second radiology information system.

### BACKGROUND OF THE INVENTION

In the field of diagnostic radiographic imaging radiology information systems (RIS) are used for managing medical patient data and image related data. Such systems can be used for defining radiology imaging orders. They commonly also comprise billing information. These systems are often used in connection with a Picture Archiving System (PACS) to manage image archives, for record keeping and for billing.

Radiographic information systems commonly have internal procedure definitions.
The following items can e.g. be comprised in a procedure definition: type of scan (CT, MR ...), contrast media to be applied / not to be applied, body part (head, thorax ..), department in the hospital, radiologist, post-processing to be applied to the image, billing information ...
These data are commonly un-structured data in a string format.

Procedure definitions depend on the specific radiology information system by means of which they are generated, a specific proprietary vocabulary is used in each system and may differ from one system to another.

Different radiology information systems may thus have different procedure definitions using different terminology for the same items.

When a hospital thus changes from one radiology information system to another, e.g. from a first system to Agfa's Enterprise Imaging System, there might be a problem because the procedure definitions in both systems may not be identical and can thus not be interpreted in a unique way.
Also in other circumstances this may occur, e.g. when a new modality is put into use or when a department or even when a whole hospital site is added to the system, e.g. to Agfa's Enterprise Imaging System.
Seamless interchanging different radiography information systems between hospitals or departments may cause a problem of procedure definition interpretation.

One way of solving this is to perform a manual table-based letter string matching of terminology, i.e. manually going through lists of procedure definitions in the first system and mapping these onto procedure definitions in the second system which have an identical meaning although they might use different terminology.

It is further possible to perform a computer implemented method based on a string search and matching process among the vocabulary (or part thereof) of both procedure definitions in order to find corresponding items.

In both cases the job is time-consuming.

Moreover, since the number of items may be large (in some cases about 10.000 items) items can be mis-labelled or missed during the mapping procedure, sometimes duplicates are present etc. In the state of the art this problem is solved by means of a matching procedure based on the bag of words representation. Vocabulary used in procedure descriptions in both systems is represented as a bag of words representation and matching algorithm is used to map the bags of words.

It is an aspect of the present invention to enhance the performance of this type of mapping method.

### Description

The invention provides a computer-implemented method which, given a set of (internal) procedure definitions in a first radiology information system generates the best match for a procedure definition defined in a second system.

The method basically tries to find similar documents from a catalog in a given radiology information system for a given input document generated in another radiology information system.

The high-level workflow of the algorithm is as follows:
Given a first procedure definition e.g. in a first radiology information system of a hospital or department, the algorithm returns the best matching procedure definition from a catalog of procedure definitions as defined in a second radiology information system.

The match is defined as a score from 0 to 1, with 1 being a perfect match.

The matching score is computed as the cosine between two vectors, one vector representing the first procedure definition, e.g. in a client system and the other representing a procedure definition from a catalog of definitions generated in a second radiology information system.

To compute the vector representation, first each procedure definition is converted to a set of tokens.

Preferably the following steps are implemented:
- Extract relevant fragments of text from various sources such as the name, code, modality, body part of the procedure definition.
- Convert to lower case
- Apply string substitutions to standardize the text, e.g. to map synonyms, fix typos, replace special characters, etc.
- Split the text into tokens based on a set of delimiters including <space> and a set of configurable characters, e.g. /, -, etc.
- Stemming and lemmatization,
- Clean and simplify tokens, e.g. by removing non-alphanumeric characters, remove vowels in large words, etc.
- Remove duplicate tokens.

Extraction of relevant fragments and splitting into tokens are mandatory steps, others are preferred embodiments.

All tokens from all first procedure definitions are gathered into a vocabulary. This vocabulary represents a multi-dimensional space where each token represents one dimension. Thus by looking up the index in the vocabulary, a dimension can be assigned to each token.

According to this invention, at least one token is also be assigned a weight. By default, every token has the same weight of 1. Certain tokens may receive a different value when they are recognized as special concept, such as modality, laterality, contrast modifier or number of views. This allows the host to give more or less weight to specific concepts, e.g. making a modality much more important by increasing its weight, or reducing the relevance for the number of views. The weight of a token can also be modified depending on the source that it was extracted from, e.g. a modality extracted from the procedure definition name vs the modality from its metadata.

In a specific embodiment, a weight is set to a value greater than 1 for a token that represents one of a modality, laterality, contrast modifier or number of views.

It is also possible that the weight is smaller than one in case of tokens that have less importance in the matching process.

In a specific embodiment, weights can also be calculated by means of training data so that the algorithm does not need manually determined substitution values.

Given its set of tokens, a procedure definition can now be written as a vector where each token represents a dimension and the coefficient for that dimension is the token's weight. Note that due to the size of the vocabulary, these vectors are very sparse as most of the coefficients are 0.

### Example:

Below is the vector representation for a catalog of two vectors defined in a first radiology information system, i.e. CT brain and MR head with tokens 'ct', 'brain', 'modality' and 'head' and wherein 'modality' is considered twice as important as other tokens:
- Vocabulary is ct, brain, mr, head
- First (in a first system) procedure definition CT brain is represented by the vector (2,1,0,0)
- First (in a first system) procedure definition MR head is represented by the vector (0,0,2,1)
- Second (in a second system) procedure definition CT head tilted is represented by the vector (2,0,0,1)

A matching algorithm is then applied to match a procedure definition in one radiology information system with a procedure definition out of the set of procedure definitions generated by the second system.

Such a matching algorithm is e.g. a matching algorithm that works according to vector cosine similarity.

The algorithm can be requested to return the top results for the best matches, not just the single best match. In case there are multiple results with the same score, it will return all results with the same score.

So, for example:
- Given a catalog of 5 first procedures, part of the vocabulary of a second radiology information system, and one second procedure, part of a different first radiology information system,
- The matching scores are 90%, 80%, 70%, 70%, 50%.
- When requesting the best result, the algorithm will return the internal procedure definition for which the matching score is 90%
- When requesting the 2 best results, it will return 2 results, those for a score of 90% and 80%
- When requesting the 3 best results, it will return 4 results, those for a score of 90%, 80%, 70% and 70%, because the 3th and 4th results have the same score.

Having described in detail preferred embodiments of the current invention, it will now be apparent to those skilled in the art that numerous modifications can be made therein without departing from the scope of the invention as defined in the appending claims.

## Claims

1. A computer-implemented method of matching a procedure definition formulated in a first Radiology Information System (client RIS) to a procedure definition in a catalog of procedure definitions defined in a second Radiology Information System (vendor RIS) by
- Generating a set of procedure definitions defined in said second RIS as a set of multidimensional vectors, each dimension of such a vector representing a token in said procedure definition, a token corresponding with a word of a vocabulary of relevant words for said procedure definition,
- Representing a procedure definition of said first RIS to be matched by a multidimensional vector, each dimension of said vector representing a token in said procedure definition, a token corresponding with a word of a vocabulary of relevant words for said procedure definitions,
- Applying to a matching algorithm to the vectors so as to generate a matching result.

2. A method according to claim 1 wherein a weight is given to at least one of said tokens.

3. A method according to claim 1 wherein said matching algorithm is based on vector cosine similarity.

4. A method according to claim 2 wherein a weight is set given to a token that represents one of a modality, laterality, contrast modifier or number of views.
